# EUROPÄISCHE PATENTSCHRIFT

(11) **EP 1 921 935 B1**
(45) Veröffentlichungstag und Bekanntmachung des Hinweises auf die Patenterteilung: **25.02.2009**
(21) Anmeldenummer: 06762408.0
(22) Anmeldetag: 05.07.2006
(51) Int. Cl.: A41B 11/00, A61F 13/06

(54) **SOCKE**
SOCK
CHAUSSETTE

(30) Priorität: 09.07.2005 DE 102005032189
(43) Veröffentlichungstag der Anmeldung: 21.05.2008
(73) Patentinhaber: X-Technology Swiss GmbH, 8832 Wollerau (CH)
(72) Erfinder: LAMBERTZ, Bodo, W., CH-8808 Pfäffikon (CH)
(74) Vertreter: Dörner, Lothar
(86) Internationale Anmeldenummer: PCT/EP2006/006537
(87) Internationale Veröffentlichungsnummer: WO 2007/006462

(56) Entgegenhaltungen:
- EP-A2- 0 499 710
- EP-A2- 1 031 291
- WO-A-99/18896
- DE-U1- 29 715 762
- US-A- 5 891 073
- US-A1- 2003 230 121

## Beschreibung

Die Erfindung betrifft eine Socke, insbesondere zum Einsatz bei sportlichen Aktivitäten, mit einem Schaft und einem Fußteil, das einen Zehen- und einen Fersenbereich und einen zwischen Zehen- und Fersenbereich gelegenen Auftrittsbereich aufweist, und die mit einer O-Ring-Bandage versehen ist.

Insbesondere bei sportlichen Aktivitäten sind die menschlichen Füße in der Regel von Socken umgeben. Um eine gute Passform der Socke am Fuß zu erzielen, ist es bekannt, O-Ring-Bandagen an Socken oder Strümpfen vorzusehen (vgl. bspw. US 5 617 745). Diese sind jedoch parallel zur Längsmittellinie der Socke symmetrisch umlaufend ausgebildet.

WO-A-99 18896 offenbart eine medizinische Knöchelbandage und EP-A-0 499 710 eine Sprunggelenkbandage.

Bei Lauf- und Sprungbewegungen kommt es zu einer erhöhten Belastung des Fußes, insbesondere im Bereich des Sprunggelenkes. Der Fuß hat die natürliche Funktion, zur Dämpfung eines Aufpralls nach innen einzuknicken. Diese Funktion wird Pronation genannt. Nach dem Aufsetzen mit der Außenseite der Sohle verlagert sich die Belastung etwas zur Innenseite, damit das Längsgewölbe des Fußes einsinken und damit einen Teil des Aufpralls absorbieren kann. Der menschliche Fuß kann jedoch unterschiedlich ausgebildet sein. Es wird unterschieden in Normalfuß, Hohl- oder Sichelfuß sowie Senkfuß. Der Normalfuß zeigt ein ausgewogenes Fußgewölbe. Er berührt beim Gehen und Laufen zuerst mit der Außenseite des Rückfußes den Boden. Dann rollt er nach innen ab, um den Aufprall des Fußes aufzufangen und zu dämpfen. Dies wird als natürliche Pronation bezeichnet. Hohl- und Sichelfüße knicken in der Landephase überwiegend nicht nach innen ab und hinterlassen überwiegend im Vor- und Rückfußbereich einen Abdruck. Dies wird als Unterpronation bzw. Supination bezeichnet. Der natürliche Aufprallschutz des Fußes ist bei der Unterpronation stark vermindert. Senkfüße hingegen haben ein sehr niedriges Fußgewölbe und hinterlassen einen kompletten Fußabdruck. Senkfüße knicken nach der Aufsetzphase sehr stark zur Innenseite ab. Dies wird als Überpronation bezeichnet. Außerdem kann der Bewegungsapparat im Bereich der Fußgelenke durch Fehlstellungen der Beine, die üblicher Weise als "O-" bzw. "X-Beine" bezeichnet werden, belastet.

Sowohl Über- als auch Unterpronierer als auch Menschen mit Fehlstellungen der Beine leiden unter dem geringen Maß an natürliche Dämpfung. Hierdurch wird der Fuß besonders belastet. Um die Sehnen und Bänder des Bewegungsapparates um das Sprunggelenk zu stützen, ist es bekannt, den Fuß zu bandagieren. Hierbei wird eine Bandage horizontal im unteren Bereich des Schienbeins um den Knöchel gewickelt, bevor die Socke über den Fuß gestülpt wird. Diese Wicklung bietet zum einen nur eine unzureichende Stabilisierung und Stützung des Bewegungsapparates um das Sprunggelenk, zum anderen wird den speziellen Belastungsformen.bei Über- bzw. Unterpronation nicht Rechnung getragen. Darüber hinaus trägt die Bandage unter der Socke sehr auf, wodurch der Tragekomfort gemindert ist.

Hier will die Erfindung Abhilfe schaffen. Der Erfindung liegt die Aufgabe zugrunde, eine Socke zu schaffen, die den Bewegungsapparat im Bereich des Sprunggelenkes stützt und dabei speziell für die besonderen Belastungen bei Über- bzw. Unterpronation sowie einer Fehlstellung der Beine ausgelegt ist. Gemäß der Erfindung wird diese Aufgabe dadurch gelöst, dass im Bereich des Sprunggelenkes mindestens eine O-Ring-Bandage angeordnet ist, die asymmetrisch auf dem Umfang der Socke verläuft.

Mit der Erfindung ist eine Socke, insbesondere für sportliche Aktivitäten geschaffen, die den Bewegungsapparat im Bereich des Sprunggelenkes stützt und speziell für die besonderen Belastungen bei Über- bzw. Unterpronation oder Fehlstellungen der Beine ausgelegt ist. Aus diesem Grund verläuft die 0-Ring-Bandage im Bereich des Sprunggelenks, um eine auf den jeweiligen Belastungsfall abgestimmte Stützfunktion erzielen zu können.

In Ausgestaltung der Erfindung verläuft die asymmetrische O-Ring-Bandage auf der Fußinnenseite unterhalb und auf der Fußaußenseite oberhalb des Knöchels. Hierdurch wird das Fußgelenk besonders gegen starke Krafteinwirkung nach innen gestützt.

In anderer Ausgestaltung der Erfindung verläuft die asymmetrische O-Ring-Bandage auf der Fußinnenseite oberhalb und auf der Fußaußenseite unterhalb der Knöchels. Hierdurch wird das Fußgelenk besonders gegen starke Krafteinwirkung nach außen gestützt.

In Weiterbildung der Erfindung sind zwei O-Ring-Bandagen vorgesehen. Hierdurch ist die auf das Sprunggelenk ausgeübte Stützfunktion zusätzlich erhöht. Durch eine gegenläufige Anordnung der Bandagen ist zudem eine beiderseits des Sprunggelenks wirkende Stützfunktion hervorgerufen.

In Ausgestaltung der Erfindung weist die Socke mindestens einen Klimakanal auf. Der Klimakanal dient der Klimaoptimierung des Fußes durch Abführung von Schwitzfeuchte. Um einen übermäßigen Materialauftrag zu vermeiden, ist der Klimakanal bevorzugt durch die Bandagen geführt.

Andere Weiterbildungen und Ausgestaltungen der Erfindung sind in den übrigen Unteransprüchen angegeben. Ausführungsbeispiele der Erfindung sind in Zeichnungen dargestellt und werden nachfolgend im Einzelnen beschrieben. Es zeigen:
- Fig. 1: eine Socke mit asymmetrischer O-Ring-Bandage in der Ansicht der Fußaußenseite;
- Fig. 2: die Darstellung des Sockenpaares nach Figur 1 in der Ansicht von hinten;
- Fig. 3: eine Socke mit asymmetrischer O-Ring-Bandage in einer anderen Ausbildung;
- Fig. 4: die Darstellung des Sockenpaares nach Figur 3 in der Ansicht von hinten;
- Fig. 5: eine Socke mit asymmetrischer O-Ring-Bandage in einer weiteren Ausbildung;
- Fig. 6: die Darstellung des Sockenpaares nach Figur 5 in der Ansicht von hinten;
- Fig. 7: eine Socke mit asymmetrischer O-Ring-Bandage in einer zusätz- lichen Ausbildung und
- Fig. 8: die Darstellung des Sockenpaares nach Figur 7 in der Ansicht von hinten.

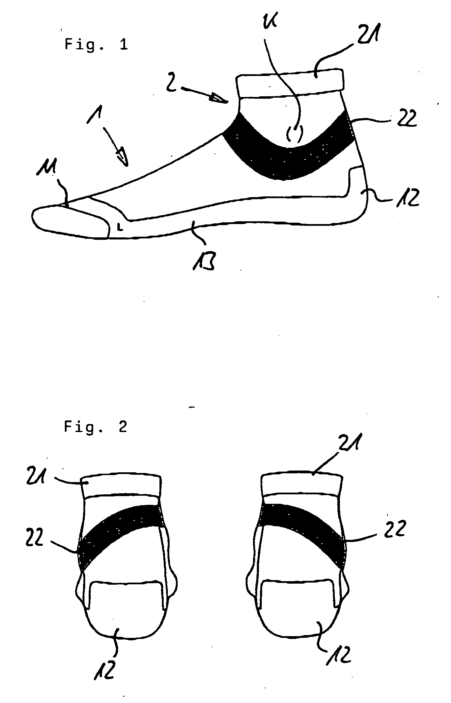

Die als Ausführungsbeispiel (Figur 1) gewählte Socke besteht aus einem Fußteil 1 und einem Schaft 2. Das Fußteil 1 weist einen Zehenbereich 11, einen ' Fersenbereich 12 und einen zwischen Zehen- und Fersenbereich gelegenen Auftrittsbereich 13 auf. Die Bereiche 11, 12 und 13 können, wie im Ausführungsbeispiel dargestellt, aus verstärktem Material hergestellt sein. Auch die Verwendung von Materialkombinationen wie beispielsweise Schurwolle mit Elastofaserwerkstoffen ist möglich.

Der Schaft 2 ist an seinem dem Fußteil 1 angewandten Ende mit einem Bund 21 versehen. Im Bereich des Sprunggelenkes ist die Socke mit einer O-Ring-Bandage 22 versehen, die asymmetrisch auf dem Umfang der Socke verläuft. Die Bandage 22 ist aus einem elastischen und auch klimaregulierenden Gewebe gebildet. Bevorzugt finden Elastan, Lycra oder andere Materialien unterschiedlicher Dehnbarkeit Anwendung.

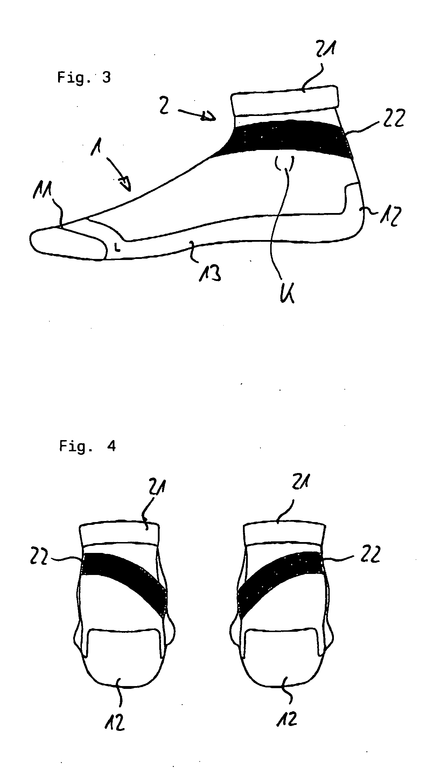

Die O-Ring-Bandage 22 ist durchgängig umlaufend und mit dem die Socke bildenden Gewebe verwoben. Dabei verläuft die O-Ring-Bandage 22 in dem Ausführungsbeispiel nach den Figuren 1 und 2 asymmetrisch auf der Fußaußenseite unterhalb des mit "K" bezeichneten Knöchels und auf der Fußinnenseite oberhalb des Knöchels. Die O-Ring-Bandage 22 stützt das Sprunggelenk sowie die um dieses angeordneten Bänder in dieser Ausführungsform besonders bei nach innen gerichteten Einwirkungen auf den Knöchel. Alternativ kann die asymmetrische O-Ring-Bandage 22 so geführt sein, dass sie - ebenfalls asymmetrisch - auf der Fußaußenseite oberhalb und auf der Fußinnenseite unterhalb des Knöchels verläuft (Figuren 3 und 4). In dieser Ausführungsform stützt sie das Sprunggelenk sowie die um dieses angeordneten Bänder besonders bei nach außen gerichteten Einwirkungen auf den Knöchel.

Bei dem in Figur 4 dargestellten Sockenpaar, bei dem die asymmetrische O-Ring-Bandage 22 derart geführt ist, dass sie auf der Fußaußenseite oberhalb und auf der Fußinnenseite unterhalb des Knöchels verläuft, ergibt sich optisch in der Ansicht von hinten eine "V-Form". Im Gegensatz hierzu ergibt sich bei dem in der Figur 2 dargestellten Sockenpaar aufgrund der Anordnung der asymmetrischen O-Ring-Bandage 22 optisch in der Ansicht der Socken von hinten gesehen eine "A-Form".

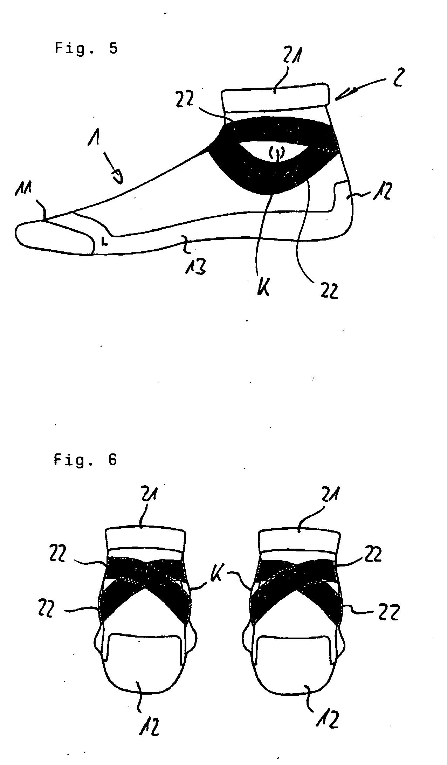

In dem in den Figuren 5 und 6 dargestellten Ausführungsbeispiel sind an der Socke zwei O-Ring-Bandagen 22 vorgesehen sein, wodurch die Stützwirkung zusätzlich erhöht ist. Es ist dabei eine gegenläufige Anordnung der Bandagen 22 gewählt, sodass sich die Bandagen 22 jeweils im Bereich der Achillessehne und dem Übergang vom Fußrücken zum Schienbein kreuzen. In der Ansicht eines Sockenpaares von hinten gesehen ergibt sich aufgrund dieser Anordnung der O-Ring-Bandagen 22 optisch eine "X-Form". Durch diese Ausführung ist eine beiderseits des Sprunggelenks wirkende Stützfunktion hervorgerufen, wobei der Knöchel "K" auf der Außen- und der Innenseite von den Bandagen 22 umgeben ist.

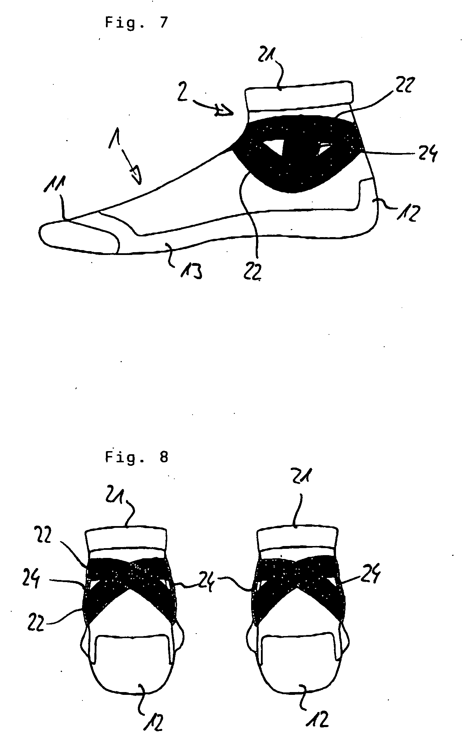

Im Ausführungsbeispiel nach den Figuren 7 und 8 sind ebenfalls zwei gegenläufige O-Ring-Bandagen 22 vorgesehen. Auf der Beinaußen- und der Beininnenseite sind die in diesem Bereich beabstandet zueinander verlaufenden Bandagen 22 durch einen Steg 24 miteinander verbunden. Die Stege 24 verlaufen über den Knöchel "K". Die Stützfunktion ist hierdurch weiter verbessert. Es ist insbesondere eine seitliche Stützfunktion hervorgerufen, bei der die Abroll- und Knickbewegung des Fußes beim Laufen oder dergleichen in keiner Weise behindert ist, da in den Bereichen, in denen der Fuß einknickt - Achillessehne und Übergang von Fußrücken zum Schienbein - die Bandagen ihre schmalste Stelle haben.

Die Socke kann mit einem - nicht dargestellten - Klimakanal versehen sein, der vom Bund 21 ausgeht und bis in den Auftrittsbereich 13 reicht und aus klimaregulierendem Netzstrickgewebe gebildet ist. Der Klimakanal trägt dazu bei, Feuchtigkeit aus dem Auftrittsbereich nach oben abzuleiten. Über dem Klimakanal ist die asymmetrische O-Ring-Bandage 22 angeordnet. Ein solcher Klimakanal kann sowohl auf der Beinaußenseite, als auch auf der Beininnenseite der Socke vorgesehen sein.

Soweit in der Beschreibung und den Ansprüchen von Socken die Rede ist, beschränkt sich die Erfindung nicht allein auf diese; vielmehr sind unter diesem Begriff auch Strümpfe, Strumpfhosen und dergleichen zu subsumieren, auf die sich die Erfindung ebenfalls bezieht.

## Patentansprüche

1. Socke, insbesondere zum Einsatz bei sportlichen Aktivitäten, mit einem Schaft und einem Fußteil, das einen Zehen- und einen Fersenbereich und einen zwischen Zehen- und Fersenbereich gelegenen Auftrittsbereich aufweist, und die mit einer O-Ring-Bandage versehen ist, **dadurch gekennzeichnet, dass** im Bereich des Sprunggelenkes mindestens eine O-Ring-Bandage (22) angeordnet ist, die asymmetrisch auf dem Umfang der Socke verläuft.

2. Socke nach Anspruch 1, **dadurch gekennzeichnet, dass** die asymmetrische O-Ring-Bandage (22) auf der Fußinnenseite unterhalb und auf der Fußaußenseite oberhalb des Knöchels verläuft.

3. Socke nach Anspruch 1, **dadurch gekennzeichnet, dass** die asymmetrische O-Ring-Bandage (22) auf der Fußinnenseite oberhalb und auf der Fußaußenseite unterhalb des Knöchels verläuft.

4. Socke nach einem der vorgenannten Ansprüche, **dadurch gekennzeichnet, dass** zwei O-Ring-Bandagen (22) vorgesehen sind.

5. Socke nach Anspruch 4, **dadurch gekennzeichnet, dass** die O-Ring-Bandagen (22) gegenläufig angeordnet sind.

6. Socke nach Anspruch 4 oder 5, **dadurch gekennzeichnet, dass** die 0-Ring-Bandagen (22) durch einen Steg (24) miteinander verbunden sind.

7. Socke nach einem der vorgenannten Ansprüche, **dadurch gekennzeichnet, dass** die Bandagen (22) aus Elastan hergestellt sind.

8. Socke nach einem der vorgenannten Ansprüche, **dadurch gekennzeichnet, dass** die Socke mindestens einen Klimakanal aufweist.

9. Socke nach Anspruch 8, **dadurch gekennzeichnet, dass** der Klimakanal aus klimaregulierendem Netzgewebe hergestellt ist.

10. Socke nach Anspruch 8 oder 9, **dadurch gekennzeichnet, dass** der Klimakanal durch die Bandagen (22) geführt ist.

## Claims

1. Sock, particularly for use in sports activities, with a leg and foot portion incorporating a toe and a heel portion and an instep portion located between the toe and heel portion, which sock is provided with an Oring bandage, **characterised in that** at least one O-ring bandage (22) running asymmetrically around the circumference of the sock is disposed in the area of the ankle joint.

2. Sock in accordance with claim 1, **characterised in that** the asymmetrical O-ring bandage (22) runs under the ankle in the inside of the foot and above the ankle on the outside of the foot.

3. Sock in accordance with claim 1, **characterised in that** the asymmetrical O-ring bandage (22) runs over the ankle in the inside of the foot and below the ankle on the outside of the foot.

4. Sock in accordance with any of the foregoing claims, **characterised in that** two O-ring bandages (22) are provided for.

5. Sock in accordance with claim 4, **characterised in that** the O-ring bandages (22) are arranged to run in opposite directions.

6. Sock in accordance with claim 4 or 5, **characterised in that** the O-ring bands (22) are connected to each other by a web (24).

7. Sock in accordance with any of the foregoing claims, **characterised in that** the bandages (22) are manufactured from elastane.

8. Sock in accordance with any of the foregoing claim, **characterised in that** the sock incorporates at least one air channel.

9. Sock in accordance with claim 8, **characterised in that** the air channel is manufactured from heat-regulating netted fabric.

10. Sock in accordance with claim 8 or 9, **characterised in that** the air channel is guided through the bands (22).

## Revendications

1. Chaussette destinée en particulier à des activités sportives, composée d'une partie montante et d'une partie pied, comportant une zone orteils et une zone talon, et une zone de déroulement du pas située entre la zone orteils et la zone talon, et dotée d'un bandage en forme joint torique, **caractérisée en ce que** dans la zone de l'articulation astragalo-calcanéenne est disposé au moins un bandage (22) en forme de joint torique présentant un tracé asymétrique sur le pourtour de la chaussette.

2. Chaussette selon la revendication 1, **caractérisée en ce que** le bandage (22) asymétrique en forme de joint torique circule en dessous de la cheville sur le côté intérieur du pied, et au dessus de celle-ci sur le côté extérieur du pied.

3. Chaussette selon la revendication 1, **caractérisée en ce que** le bandage (22) asymétrique en forme de joint torique circule au dessus de la cheville sur le côté intérieur du pied, et en dessous de celle-ci sur le côté extérieur du pied.

4. Chaussette selon l'une des revendications précédentes, **caractérisée en ce que** deux bandages (22) en forme de joint torique ont été prévus.

5. Chaussette selon la revendication 4, **caractérisée en ce que** les bandages (22) en forme de joint torique sont agencés en sens contraires.

6. Chaussette selon les revendications 4 ou 5, **caractérisée en ce que** les bandages (22) en forme de joint torique sont reliés entre eux par une nervure (24).

7. Chaussette selon l'une des revendications précédentes, **caractérisée en ce que** les bandages (22) ont été confectionnés en élasthanne.

8. Chaussette selon l'une des revendications précédentes, **caractérisée en ce que** la chaussette comporte au moins un canal climatique.

9. Chaussette selon la revendication 8, **caractérisée en ce que** le canal climatique a été fabriqué en un textile réticulaire régulateur du climat.

10. Chaussette selon la revendication 8 ou 9, **caractérisée en ce que** le canal climatique est guidé par les bandages (22).
